# EUROPEAN PATENT SPECIFICATION

(11) **EP 0 812 179 B1**
(45) Date of publication and mention of the grant of the patent: **07.04.2004**
(21) Application number: 96901047.9
(22) Date of filing: 26.01.1996
(51) Int. Cl.: A61K 6/00

(54) **ADHESIVE FOR DENTAL PROSTHESES**
HAFTMITTEL FÜR ZAHNPROTHESEN
ADHESIF POUR PROTHESES DENTAIRES

(30) Priority: 27.02.1995 GB 9503905
(43) Date of publication of application: 17.12.1997
(73) Proprietor: Kukident GmbH, D-69469 Weinheim (DE)
(72) Inventor: VAN DE LÖCHT-BLASBERG, Monika, D-69469 Weinheim (DE); KNOLLMAN, Rainer, D-32052 Herford (DE)
(74) Representative: Hall, Marina
(86) International application number: PCT/GB1996/000164
(87) International publication number: WO 1996/026704

(56) References cited:
- WO-A-92/10988
- DE-A- 4 124 050
- US-A- 4 894 232
- US-A- 4 981 875

## Description

The invention relates to a novel composition for use as an adhesive for dental prosthesis, the use thereof and a process for the preparation of a medicament for preventing or alleviating inflammation in the oral mucosa region.

Although, it is known that adhesives per se fulfil a preventative function, because they form a film on the prosthesis and in this way protect the sensitive mucosa against pressure points and inflammation, in the case of dental prosthesis wearers there are still pressure points and inflammation in the oral mucosa region. Thus, for reinforcing the preventative action there are prosthesis adhesives on the market, which contain as the antiinflammatory active substance ∝-bisabolol which may be derived from camomile.

DE 41 24 050 A1 is directed to a pharmaceutical composition on-the basis of a dry camomile extract which is enriched in matricine to obtain a stronger antiphlogistic effect.- Specific process of drying and extracting camomile blossoms to obtain such extract is disclosed.

US-A-4,981,875 is directed to medicaments for the region of the oral cavity containing etofenamate as the main active agent. Such compositions may additionally contain camomile extract.

An aim of the present invention is to make available a prosthesis adhesive with improved antiphlogistic characteristics.

To this end, according to the invention there is provided an oral adhesive composition.in the form of a cream, ointment or gel for use as an adhesive for dental prosthesis, comprising :
i) 30 to 60 wt.% of at least one active adhesive substance,
ii) 30 to 69 wt.% of a base for the cream, ointment or gel, and
iii) 1 to 15 wt.% of a camomile extract comprising antiphlogistically active camomile constituents and which is compatible with said base.

Preferably, the composition according to the invention has a content of camomile extract of 1 to 10 wt.%, based on a camomile extract-in a preparation with approximately 10 % camomile constituents.

Optionally, the composition comprises up to 2 wt% of further additives such as flavouring agents, dyes and stabilizers.

Preferably the base for the cream, ointment or gel in the composition comprises a substance chosen from the group constituted by paraffin oil, sunflower oil, 'soy oil (that is to say soya bean oil), petroleum jelly (Vaseline) and hydrophobic oleogels.

It is preferably for the active adhesive substance in a composition according to the invention to be selected from the group comprising-sodium carboxymethyl cellulose, composition according to the invention to be selected from the group comprising sodium carboxymethyl cellulose, sodium alginate, copolymer salts of methyl vinyl ether/maleic anhydride, polyvinyl acetate., polyvinyl pyrrolidone, hydroxyethyl cellulose, polyoxymethylene, polyacrylamides and mixtures thereof.

A composition according to the invention may contain at least one further addition of an antiphlogistically active substance, such as ∝-bisabolol.

Further according to the invention there is provided a composition of the invention being a medicament for preventing or alleviating inflammation in the oral mucosa region, in which camomile constituents are mixed with at least one active adhesive substance.

It has surprisingly been found that the dental prosthesis compositions according to the invention have a better antiphlogistic action than the known adhesives which include only one constituent of camomile, ∝-bisabolol. Although the applicant does not wish to be bound by it, the hypothesis can be made that there is a synergistic action of the main constituents in the camomile constituents, which surprisingly leads to said constituents being antiphlogistically more active than ∝-bisabolol alone. The main camomile constituents, in addition to the ∝-bisabolol, which could play a further part here are azulenes (chamazulene, guajazulene), further sesquiterpene derivatives (bisabolol oxides A, B and C, bisabolone oxide A), and flavone glycosides (hyperoxide, rutin, luteolin-7-glucoside, apigenin-7-glucoside), cumarin and farnesene.

In addition, in a confidential consumer test it was found that unexpectedly the adhesive composition according to the invention with camomile constituents was also better evaluated with respect to adhesion than other products. Finally, the superior drawability of the product according to the invention was established, which also indicates improved adhesion..

The invention is described in greater detail hereinafter by the following examples, which are in.the form of preparation examples and test results concerning the anti-inflammatory/inflammation-preventing activity, as well as the improved adhesion of the adhesive according to the invention. In the drawings the figures are as follows:
- Fig.1: A graph representing the capillary cutaneous circulation in the case of three adhesive creams according to the invention and untreated skin surface (control), plotted against time.
- Fig.2: A graph showing the capillary cutaneous circulation in the preferred adhesive cream according to the invention (HM 239), two different comparison substances (HP1 and HP2) and the untreated skin surface (control), plotted against time.
- Fig.3: A diagram showing the confidential consumer test results with respect to the purchasing readiness of a product according to the invention and two comparison products.
- Fig.4: A diagram showing the confidential consumer test results with regards to the product performance overall for.a product according to the invention and two comparison products.
- Fig.5: A diagram showing the confidential consumer test results relative to the general evaluation of a product according to the invention and two comparison products.
- Fig.6: A diagram showing the confidential consumer test results with respect to the adhesive action of a product according to the invention and two comparison products.
- Fig.7: A diagram comparing the drawability (in Newtons) for a product according to the invention and two comparison products.

Before going into detail on the different exemplified formulations, we wish to point out that in general any camomile constituents are suitable within the scope of the present invention which have an antiphlogistic action, independently of the extracting agent and the camomile species (growing, part of the plant, cultivation area, etc.).

Hydrophilic solvents (alcohols, glycols) of the cream, gel or ointment may be removed and the preparation carried out with more hydrophobic and therefore more oil-compatible solvents, such as e.g. soy oil (soya bean oil) or sunflower oil.

In the following examples, the camomile extract used in Example 1 is Phytoconcentrol Camomile Oil-Soluble 2/066310 and that used in Example 2-is Neo-Extrapon Camomile 2/060350 (both from Dragoco). Other suitable extracts in oil-compatible preparation are e.g. camomile extracts LS 2416 G (soy oil ie soya bean oil), LS 2904 G (sunflower oil) or LS 3066 G (diisobutyl adipate) from Grau Aromatics (or corresponding products from Gattefossé GmbH e.g. Vegetol 4140).

### Example 1: Preparation of an adhesive cream according to the invention

The following tables 1 and 2 give exemplified formulations for an adhesive cream with camomile extract, the products with the designations VM 196, HM 239 and HM 347 are in the particularly preferred range for the camomile extract content. The given formulations were used for the subsequently described tests. The camomile extract used was present in a preparation with approximately 10% camomile constituents.

**TABLE 1**

| | | |
|---|---|---|
| Constituents | VM 196 | HM 239 |
| Paraffin oil | 31.88 | 32.88 |
| Vaseline | 12.00 | 12.00 |
| Peppermint oil | 0.10 | 0.10 |
| Menthol | 0.02 | 0.02 |
| Camomile extract (oil-soluble) | 3.00 | 2.00 |
| Sodium carboxymethyl cellulose (high viscosity) | 15.00 | 15.00 |
| Sodium carboxymethyl cellulose (medium viscosity) | 8.00 | 8.00 |
| Poly(maleic acid/methyl vinyl ether) calcium / sodium salt | 30.00 | 30.00 |

**TABLE 2**

| | | |
|---|---|---|
| Constituents | HM 347 | HM 349 |
| Paraffin oil | 30.375 | 32.075 |
| Vaseline | 14.50 | 13.80 |
| Peppermint oil | 0.10 | 0.10 |
| Menthol | 0.02 | 0.02 |
| Dye E 127 | 0.005 | 0.005 |
| Camomile extract (oil-soluble) | 2.00 | 1.00 |
| Sodium carboyxmethyl cellulose (high viscosity) | 15.00 | 15.00 |
| Sodium carboxymethyl cellulose (medium viscosity) | 8.00 | 8.00 |
| Poly(maleic acid/methyl vinyl ether) calcium sodium salt | 30.00 | 30.00 |

The preparation of the corresponding adhesive creams in each case took place in the following way.

A Unimix mixer was pre-heated to 38°C. On reaching the temperature the weighed quantities of paraffin oil, melted Vaseline and optionally dye were fed in. This was followed by the addition of a mixture, prepared on the previous day, of peppermint oil and menthol, as well as the camomile extract. The mixture was then stirred for 3 minutes at 50 r.p.m.

This was followed by the addition of the pulverulent adhesive raw materials by filling connection and accompanied by stirring in the following order: poly(maleic acid/methyl vinyl ether) calcium sodium salt, sodium carboxymethyl cellulose (high viscosity) and sodium carboxymethyl cellulose (medium viscosity).

After adding all the powder components the mixture was stirred for a further 60 minutes at 50 r.p.m. until all the constituents were homogeneously distributed.

### Example 2: Testing the anti-inflammatory/inflammation inhibiting activity of the adhesive of the invention

For the activity test described in greater detail hereinafter comparison took place between the adhesive creams prepared according to example 1, a standard commercial product (HP 1), a commercial product with α-bisabolol (HP 2) and the untreated skin surface (control).

Testing took place on volunteer test persons, under secrecy provisions, with a healthy skin. Using a solar simulator, irradiation took place until the erythema threshold was reached. Immediately following radiation the previously divided up test areas were treated with the formulations (control: untreated).

Application of the test preparations took place under occlusion, in order to come close the conditions of the oral mucosa. A subsequent treatment took place after 3, 6, 9, 24 and 48 hours.

The antiphlogistic activity was tested by measurements of capillary cutaneous circulation using the laser-Doppler flow meter. The lower the cutaneous circulation values the lower the erythema/irritation effects and the higher the antiphlogistic activity of the test product.

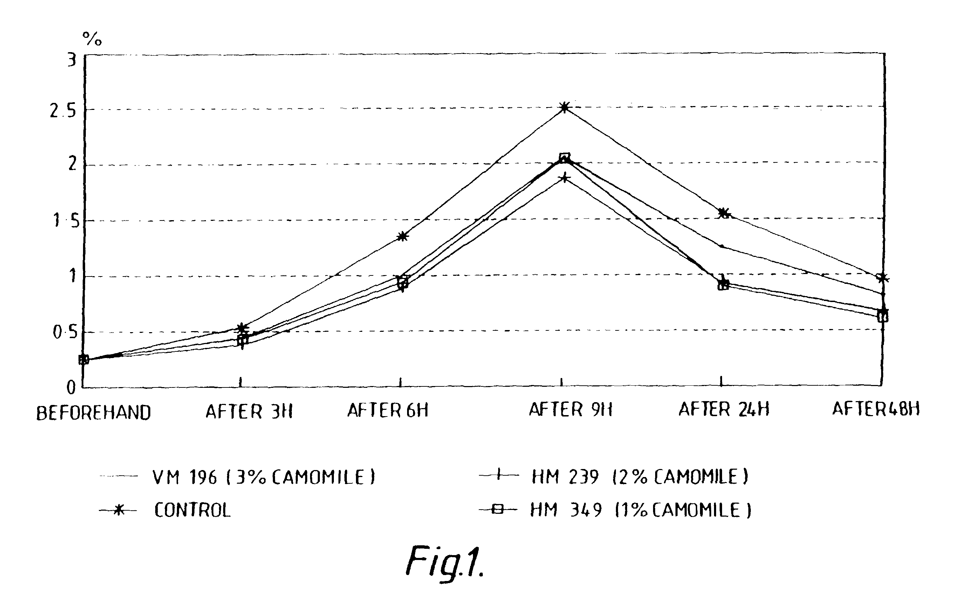

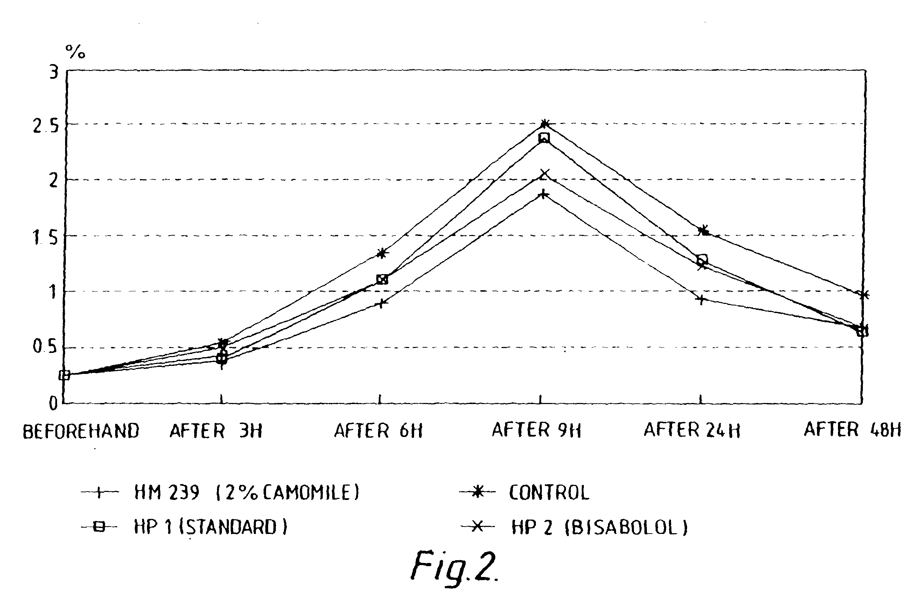

This process has been established as an investigation model for determining the antiphlogistic activity. It is possible to transfer the results from the normal skin to the oral mucosa, because as a result of easier resorption conditions in the oral mucosa, a reinforcement of the antiphlogistic properties is to be expected. The test results are compared in figs. 1 and 2.

Fig. 1 shows that even a 1% camomile extract content reveals a superior action compared with the control, but that an even more pronounced improvement results from increasing the content to 2%.

A further increase in the camomile content to 3% leads to no further improvement of the antiphlogistic activity and in fact causes a slight deterioration compared with the product having a 2% camomile extract content.

Fig. 2 compares the untreated skin surface (control), products HP 1 and HP 2 and the product according to the invention with the optimum camomile extract content. The superior action of the adhesive according to the invention is very apparent.

### Example 3: Testing the adhesion of the adhesive cream according to the invention

In the following tests comparisons took place between the standard adhesive cream product (HP 1) known from example 2, the commercial product with α-bisabolol (HP 2) known from example 2 and the product according to the invention (VP) (corresponding to product HM 239 of example 1) in a concealed consumer test and in a drawability test.

The confidential consumer test was carried out in such a way that the test products were concealed and in monadic manner by the consumer at home. The consumer was given brief instructions for use. Each group consisted of 60 persons, all of whom were adhesive cream or adhesive gel users.

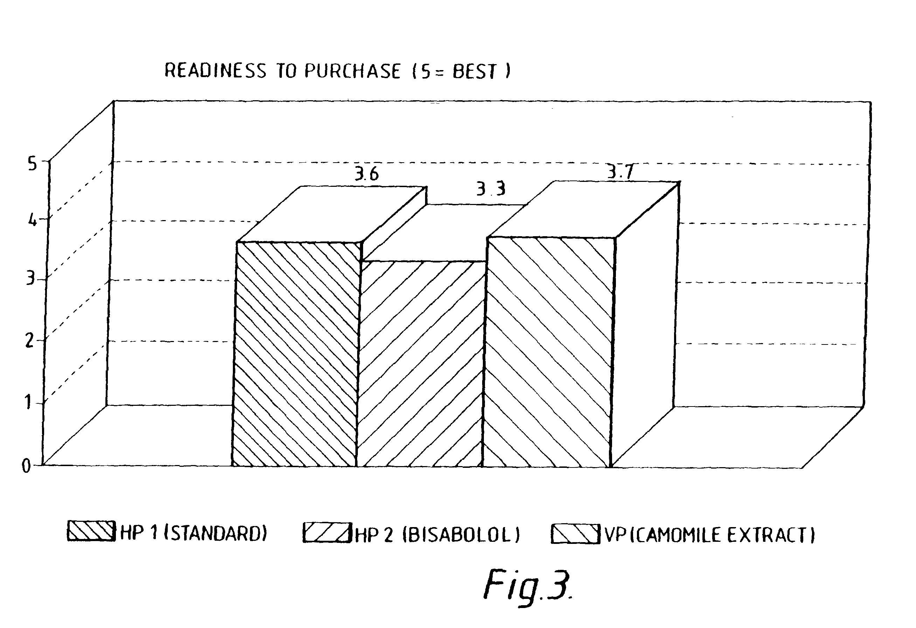

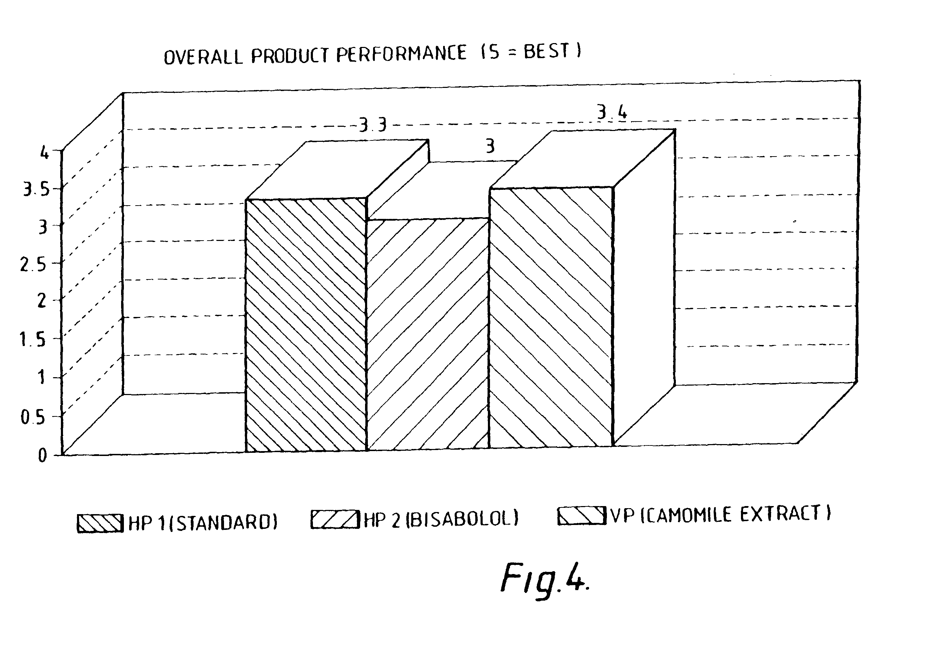

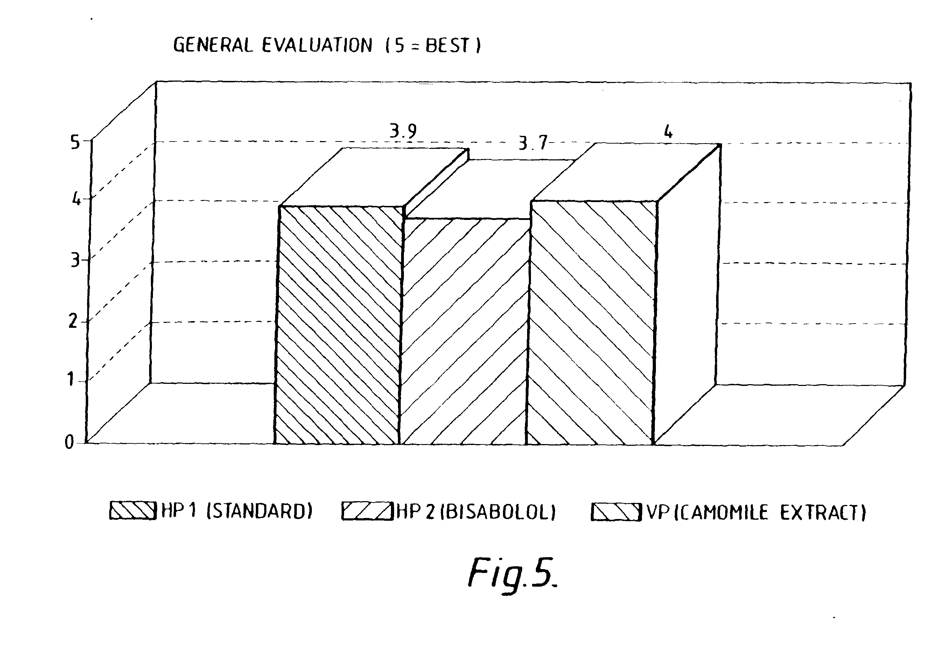

The evaluation scale extended from 1 to 5 and 5 represented the maximum number of points attainable. The results are shown in figs. 3 to 6 in the categories readiness to purchase, product performance overall, general evaluation and adhesive action.

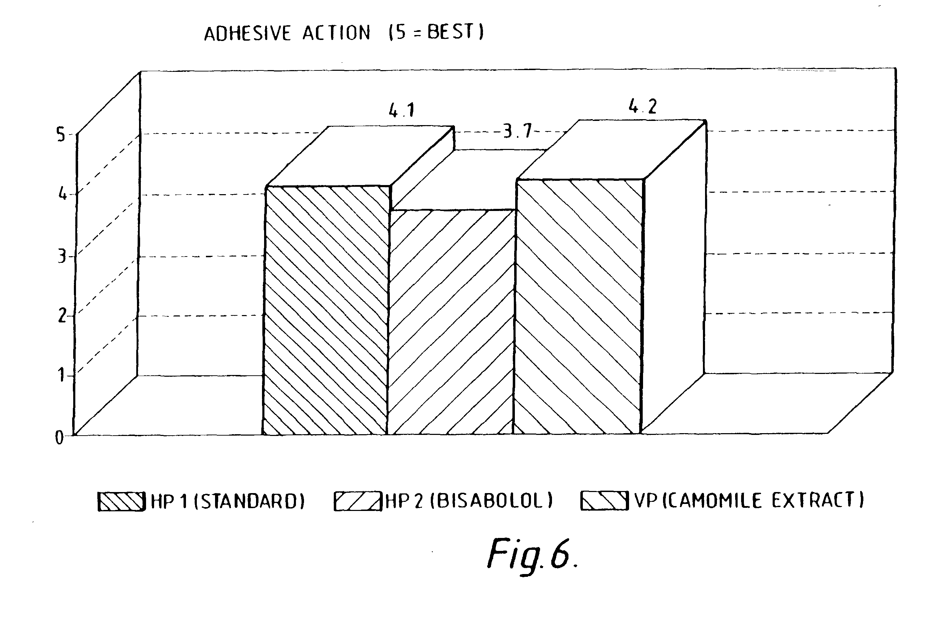

The product according to the invention with camomile extract revealed superior values in all categories. of greatest interest are the adhesive action results of fig.6.

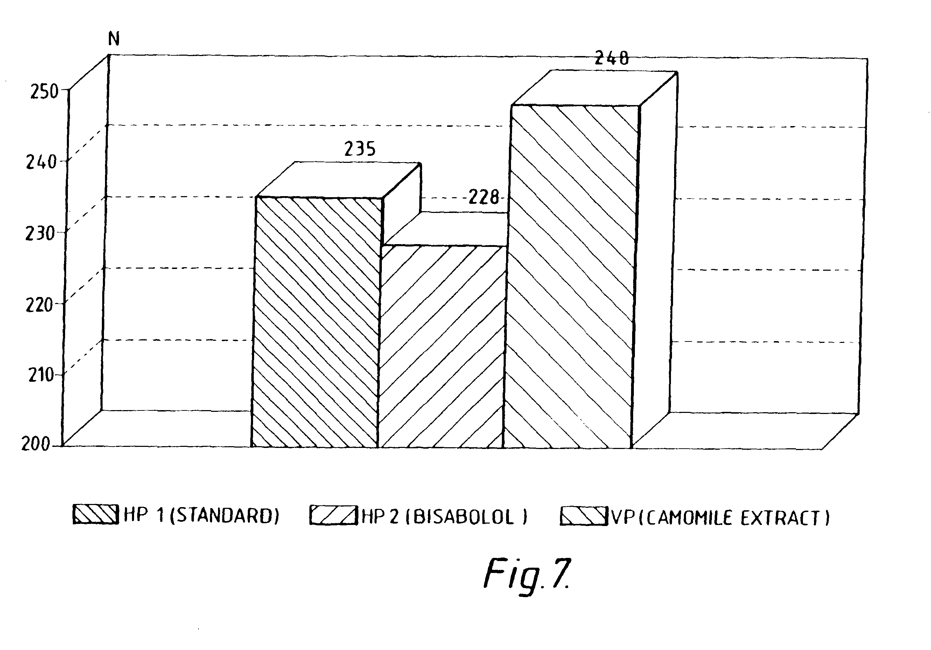

Fig. 7 compares the results of drawability test for the three aforementioned products. The test was performed in such a way that the test substance was placed on a plate, comprising a commercial prosthesis plastic material, weighed in and mixed with a clearly defined quantity of water. This place was screwed into the tensile and compressive force measuring instrument (Erichsen 391). At the top was also screwed in a plate made from a commercial prosthesis plastic material, but which was additionally provided with an underlining material support which remained soft. The two plates were then compressed under clearly defined conditions.

After a clearly defined time the force (in N) was measured, which was necessary for pulling apart the two plates. The read off value represents the drawability in N.

## Claims

1. An oral adhesive composition in the form of a cream, ointment or gel for use as an adhesive for dental prosthesis, comprising :
i) 30 to 60 wt.% of at least one active adhesive substance,
ii) 30 to 69 wt.% of a base for the cream, ointment or gel,
and
iii) 1 to 15 wt.% of a camomile extract comprising antiphlogistically active camomile constituents and which is compatible with said base.

2. A composition according to claim 1, in which the content of camomile extract is 1 to 10 wt.%, based on a camomile extract in a preparation with approximately 10 % camomile constituents.

3. A composition according to claim 1 or 2, comprising up to 2 wt.% of further additives such as flavouring agents, dyes and stabilizers.

4. A composition according to any preceding claim, in which the base for the cream, ointment or gel is chosen' from the group comprising paraffin oil, sunflower oil, soy oil (soya bean oil), petroleum jelly and hydrophobic oleogels.

5. A composition according to any preceding claim, in which the active adhesive substance is selected from the group comprising sodium carboxymethyl cellulose, sodium alginate, copolymer salts methyl vinyl ether/maleic anhydride, polyvinyl acetate, polyvinyl pyrrolidone, hydroxyethyl cellulose, polyoxymethylene, polyacrylamides and mixtures thereof.

6. A composition according to any preceding claim, in which the composition further contains at least one further antiphlogistically active substance, preferably ∝-bisabolol.

7. A process for the preparation of a composition according to any of claims 1 to 6, being a medicament for preventing or alleviating inflammation in the oral mucosa region, **characterized by** the step of mixing camomile constituents with at least one active adhesive substance.

## Patentansprüche

1. Orale Haftzusammensetzung in Form einer Creme, einer Salbe oder eines Gels zur Verwendung als Haftmittel für Dentalprothesen, umfassend:
i) 30 bis 60 Gew.-% mindestens einer aktiven Haftsubstanz,
ii) 30 bis 69 Gew.-% einer Basis für die Creme, die Salbe oder das Gel, und
iii) 1 bis 15 Gew.-% eines Kamillenextrakts, der entzündungshemmende aktive Kamillenbestandteile umfasst und mit der Basis kompatibel ist.

2. Zusammensetzung nach Anspruch 1, in der der Gehalt des Kamillenextrakts 1 bis 10 Gew.-% beträgt, basierend auf einem Kamillenextrakt in einer Zubereitung mit ca. 10 % Kamillenbestandteilen.

3. Zusammensetzung nach Anspruch 1 oder 2, umfassend bis zu 2 Gew.-% weiterer Zusätze, wie Geschmacksstoffe, Farbstoffe und Stabilisatoren.

4. Zusammensetzung nach einem der vorstehenden Ansprüche, in welcher die Basis für die Creme, die Salbe oder das Gel ausgewählt wird aus der Gruppe umfassend Paraffinöl, Sonnenblumenöl, Sojaöl (Sojabohnenöl), Vaseline und hydrophoben Oleogelen.

5. Zusammensetzung nach einem der vorstehenden Ansprüche, in welcher die aktive Haftsubstanz ausgewählt ist aus der Gruppe umfassend Natriumcarboxymethylcellulose, Natriumalginat, Copolymersalzen-Methylvinylether/Maleinsäureanhydrid, Polyvinylacetat, Polyvinylpyrrolidon, Hydroxyethylcellulose, Polyoxymethylen, Polyacrylamiden und Gemischen davon.

6. Zusammensetzung nach einem der vorstehenden Ansprüche, in welcher die Zusammensetzung weiterhin mindestens eine weitere entzündungshemmende aktive Substanz, vorzugsweise α-Bisabolol, enthält.

7. Verfahren zur Herstellung einer Zusammensetzung nach einem der Ansprüche 1 bis 6, wobei diese ein Medikament zur Vorbeugung oder Linderung einer Entzündung im Bereich der Mundschleimhaut ist, **gekennzeichnet durch** den Schritt des Mischens von Kamillenbestandteilen mit mindestens einer aktiven Haftsubstanz.

## Revendications

1. Composition adhésive à usage oral sous la forme d'une crème, d'un onguent ou d'un gel pour une utilisation en tant qu'adhésif pour une prothèse dentaire, comprenant :
i) 30 à 60 % en poids d'au moins une substance adhésive active,
ii) 30 à 69 % en poids d'une base pour la crème, l'onguent ou le gel, et
iii) 1 à 15 % en poids d'un extrait de camomille comprenant des constituants de camomille ayant une activité antiphlogistique et qui est compatible avec ladite base.

2. Composition selon la revendication 1, dans 'laquelle la teneur en extrait de camomille est de 1 à 10 % en poids sur la base d'un extrait de camomille dans une préparation avec environ 10 % de constituants de camomille.

3. Composition selon la revendication 1 ou 2, comprenant jusqu'à 2 % en poids d'additifs supplémentaires comme des agents aromatisants, des colorants et des stabilisants.

4. Composition selon l'une quelconque des revendications précédentes, dans laquelle la base pour la crème, l'onguent ou le gel est choisie dans l'ensemble comprenant l'huile de paraffine, l'huile de tournesol, l'huile de soja (huile de graines de soja), la vaseline et des oléogels hydrophobes.

5. Composition selon l'une quelconque des revendications précédentes, dans laquelle la substance adhésive active est choisie dans l'ensemble comprenant la carboxyméthylcellulose sodique, l'alginate de sodium, des sels de copolymères d'éther méthylvinylique/anhydride maléique, l'acétate de polyvinyle, la polyvinylpyrrolidone, l'hydroxyéthylcellulose, le polyoxyméthylène, des polyacrylamides et des mélanges de ceux-ci.

6. Composition selon l'une quelconque des revendications précédentes, ladite composition contenant, en outre, au moins une substance supplémentaire ayant une activité antiphlogistique, de préférence de l'a-bisabolol.

7. Procédé de préparation d'une composition selon l'une quelconque des revendications 1 à 6, qui est un médicament pour prévenir ou soulager une inflammation dans la région de la muqueuse buccale, **caractérisé par** l'étape consistant à mélanger des constituants de camomille avec au moins une substance adhésive active.
